Europäisches Patentamt

European Patent Office

Office européen des brevets

(11) Numéro de publication: **0 409 674 A1**

# DEMANDE DE BREVET EUROPEEN

(21) Numéro de dépôt: 90401662.3

(22) Date de dépôt: **14.06.90**

(51) Int. Cl.⁵: **A61M 5/30**

Le titre de l'invention a été modifié (Directives relatives à l'examen pratiqué à l'OEB, A-III, 7.3)

(30) Priorité: **16.06.89 FR 8908031**

(43) Date de publication de la demande:
**23.01.91 Bulletin 91/04**

(84) Etats contractants désignés:
**AT BE CH DE DK ES FR GB GR IT LI LU NL SE**

(71) Demandeur: **PASTEUR MERIEUX SERUMS ET VACCINS, Sociéte Anonyme :**

**58 Avenue Leclerc**
**F-69348 Lyon Cédex 07(FR)**

(72) Inventeur: **Galy, Michel, Germain, Henry**
**Le Blein, St.Loup**
**F-69490 Pontchara-sur-Turdine(FR)**

(74) Mandataire: **Bernasconi, Jean et al**
**CABINET LEMOINE ET LEMOINE, 13**
**Boulevard des Batignolles**
**F-75008 Paris(FR)**

(54) **Appareil d'injection sans aiguille à doses contenues dans des cartouches et cartouches à utiliser dans un tel appareil.**

(57) L'appareil d'administration par jet sans aiguille de doses contenues dans des cartouches (1) est caractérisé en ce que l'extrémité antérieure de la cartouche (4, 5) est conformée de façon à empêcher un contact de l'extrémité antérieure (20) d'un logement de cartouche avec la zone corporelle avec laquelle vient en contact l'extrémité de la cartouche et en ce que la cartouche, en polypropylène, est entourée d'une frette métallique à sa partie antérieure.

FIG.1

## PERFECTIONNEMENT AUX APPAREILS D'INJECTION SANS AIGUILLE À DOSES CONTENUES DANS DES CARTOUCHES ET CARTOUCHES À UTILISER DANS UN TEL APPAREIL.

La présente invention a trait à un perfectionnement aux appareils d'injection sans aiguille, à doses contenues dans des cartouches.

On utilise de plus en plus, pour l'administration de substances médicamenteuses par voie parentérale, et notamment pour la vaccination, des appareils d'injection sans aiguille délivrant la dose sous forme d'un jet très mince et très puissant. Ces applications se sont développées, non seulement en médecine vétérinaire, mais également en médecine humaine, l'absence d'aiguille d'injection permettant d'utiliser un personnel formé rapidement, d'augmenter les cadences et d'éviter les contaminations bactériennes et surtout virales qui se produisent dans les cas où une même aiguille est utilisée sans stérilisation pour plusieurs sujets. Cependant, si, par l'utilisation de ces appareils à injection par jet transcutané, la contamination d'un sujet à l'autre paraît exclue, il s'agit seulement d'une apparence car la déposante a constaté que la puissance du jet, au moment où il pénètre dans la peau, provoque des projections de matières organiques solides ou liquides, qui peuvent souiller la buse d'éjection de l'appareil et qui, entraînées par le jet suivant, peuvent infecter un autre personne.

Ces appareils d'injection possèdent en général une chambre susceptible d'être remplie d'une dose de médicament ou vaccin à partir d'un réservoir, chambre dans laquelle se déplace brutalement un piston qui éjecte la dose à travers une fine buse permettant l'éjection d'un jet puissant, fin et calibré. Il existe également des appareils dans lesquels la chambre et la buse sont remplacées par un logement permettant de recevoir des carpules en forme de cartouches présentant une buse calibrée à leur extrémité antérieure, et un piston postérieur emprisonnant la dose, ce piston étant frappé par une tige ou masse projetée en avant par l'appareil pour assurer l'éjection. De tels appareils sont décrits par exemple dans les brevets US-3.688.765, 4.403.609 et 4.421.508. Dans certains de ces appareils, l'extrémité anrtérieure de la cartouche, qui comporte la buse, émerge à l'avant de l'appareil lui-même, c'est-à-dire de l'extrémité antérieure du logement recevant la cartouche, de sorte que l'on aurait pu penser que de tels appareils présentent une innocuité totale quant aux risques de contamination puisque le cartouche est changé à chaque administration. Cependant, si les risques de contamination sont en fait réduits, ils sont loin d'être supprimés, en raison notamment des projections liquidiennes qui parviennent à contaminer la cartouche suivante.

Le brevet US-2.550.053 décrit un appareil d'injection par jet comprenant une cartouche dont l'extrémité antérieure émerge à l'avant de l'appareil. Les risques de contamination dans un tel appareil sont plus réduits. Cependant, ce dispositif nécessite des cartouches extrêmement résistantes, en métal, et le mécanisme électrique de propulsion, avec percuteur, permettant le déplacement du piston de la cartouche, n'est pas utilisable dans la vaccination tout terrain. En outre, ce mécanisme entraîne un choc initial important qui le rend inutilisable dans la pratique. En effet, pour assurer une administration convenable d'une dose de médicament ou de vaccin par jet transcutané, il est indispensable d'obtenir un profil de pression avec une montée brusque mais limitée à la valeur requise, un palier et une descente toute aussi brusque de façon à ce que la totalité de la dose se trouve injectée à la profondeur voulue.

Le brevet US-3.782.380 décrit également un appareil de ce type mais dans lequel la cartouche, par exemple en verre, est contenue dans un magasin qui vient en contact avec le corps. Ce magasin dans lequel on introduit puis on extrait chaque cartouche, est donc une cause de contamination. Ce brevet présente, en outre, le même inconvénient que le précédent, à savoir que le percuteur repoussé par le moteur, en l'occurence un ressort, possède d'abord une course libre avant de venir frapper le piston de la cartouche, course pendant laquelle le percuteur acquiert une vitesse intense de sorte qu'il se produit un choc extrêmement brutal au début de l'injection. Dans le cas d'une cartouche en verre, il est même peu vraisemblable que, malgré la présence du magasin, celle-ci puisse résister au choc, le magasin ne pouvant épouser étroitement la surface du verre en raison des tolérances et des nécessités de l'introduction et de l'extraction.

On connait également, d'après les brevets US-3.802 430 et 4.124.024, des dispositifs pyrotechniques avec une charge de poudre destinée à propulser un piston dans un ensemble complexe. De telles réalisations sont des vues de l'esprit et ne sont pas utilisables en pratique.

L'invention se propose de remédier à ces inconvénients et de fournir un appareil d'administration par jet sans aiguille de doses médicamenteuses, par exemple de vaccin, du type à cartouche, permettant, à prix de revient particulièrement abaissé, de supprimer le risque de contamination croisée en assurant l'injection de la totalité de la dose éjectée.

Un autre objectif de l'invention est de réaliser un tel appareil qui présente les avantages propres à l'utilisation des cartouches et notamment une sécurité du prédosage et de la stérilité de la dose.

Un autre objectif encore de l'invention est de fournir un tel appareil dans lequel une cartouche, une fois utilisée, ne puisse pas être réutilisée.

Un autre objectif encore de l'invention est de fournir un tel appareil qui soit d'un maniement simple et adapté aux conditions tout terrain.

Un autre objectif encore de l'invention est de fournir des cartouches dosées permettant d'obtenir les avantages précités.

Par appareil, dans le sens de la présente invention, on entend non seulement l'appareil chargé de sa cartouche mais également les constituants de cet appareil, à savoir l'appareil d'éjection lui-même, d'une part, et la cartouche prise séparément, d'autre part.

L'invention a pour objet un appareil d'administration par jet sans aiguille de doses contenues dans des cartouches, du type comprenant un logement destiné à recevoir une cartouche comprenant un corps muni à son extrémité antérieure d'une buse permettant la formation du jet, et vers son extrémité postérieure, un piston pour l'expulsion de la dose contenue dans la cartouche, ledit appareil comprenant un moyen moteur, par exemple à ressort mis sous tension manuellement et lançant une tige de percussion, susceptible d'être déclenché par l'opérateur pour repousser vers l'avant le piston de la cartouche et assurer la formation du jet et l'évacuation de la dose, l'appareil étant agencé pour que l'extrémité antérieure de la cartouche émerge de l'extrémité antérieure du logement de l'appareil, suffisamment pour empêcher un contact de l'extrémité antérieure dudit logement avec la zone corporelle avec laquelle vient en contact l'extrémité de la cartouche, caractérisé en ce que la cartouche, réalisée en un matériau synthétique, de préférence en polypropylène, présente une partie antérieure entourée par une enveloppe métallique épousant étroitement ladite partie de façon à empêcher sa déformation pendant l'injection.

Dans un mode de mise en oeuvre préféré de l'invention, l'appareil comporte un organe de freinage freinant le mouvement de la tige qui vient frapper le piston de la cartouche pendant la course à vide pendant laquelle l'extrémité antérieure de la tige se déplace entre sa position initiale et sensiblement la position dans laquelle elle vient au contact du piston de la cartouche.

On obtient ainsi, d'une façon remarquable, grâce à l'invention, un profil de pression dans la cartouche pendant l'injection, caractérisé par un front ascendant extrêmement raide jusqu'à la valeur désirée sans pic de pression supplémentaire tendant à déformer la cartouche, compte tenu de la présence de la frette ou enveloppe, un palier décroissant progressivement en raison de la détente progressive du ressort moteur et à front descendant tout aussi raide de sorte que toute la dose se trouve éjectée sous forme d'un jet mince perforant de puissance pratiquement constante, et ceci sans avoir, vers la fin de l'éjection, l'inconvénient d'une partie de liquide ne pénétrant pas dans la partie corporelle et venant s'interposer entre la peau et la cartouche et susceptible de provoquer des souillures éventuelles au niveau de l'extrémité antérieure de l'appareil.

On constate que l'on peut se dispenser d'entourer le corps de cartouche de l'enveloppe sur la totalité de sa longueur, la partie postérieure, transparente, permettant de voir le piston et le liquide à l'intérieur lors de la mise en place de la cartouche dans l'appareil, sans observer de déformation initiale malgré la montée rapide en pression.

Le moyen de freinage est de préférence du type hydraulique mais pourrait être de toute autre nature, par exemple à friction. Il pourrait même être porté par la cartouche elle-même, par exemple en prévoyant à la partie postérieure de la cartouche une paroi fracturable qui doit être fracturée par la tige de percussion avant que celle-ci ne puisse agir sur le piston contenu à l'intérieur de la cartouche.

La distance de la partie émergeante de cartouche, totalement revêtue de l'enveloppe, est de préférence de l'ordre de 5 mm ou davantage, de sorte que si l'on tient compte de la longueur de l'extrémité rétrécie de cartouche contenant la buse d'éjection, qui peut elle-même être de l'ordre de 5 mm, l'extrémité antérieure du logement est distante, axialement, d'une valeur de l'ordre de 10 mm ou plus, du débouché de la buse ou canal d'éjection. Du fait que le diamètre extérieur du corps de cartouche est lui-même en général de l'ordre de 10 mm ou davantage, on parvient ainsi à éliminer pratiquement tout risque de contamination d'un sujet à l'autre car ou bien le logement ne vient même pas au contact de la zone corporelle qui entoure l'emplacement de l'épiderme où se fait l'injection, ou bien le logement ne vient au contact de l'épiderme qu'à une distance très importante de l'endroit où peuvent exister des projections de matières corporelles.

De préférence, l'enveloppe présente une partie cylindrique entourant ladite partie antérieure du corps et une partie radiale venant recouvrir le raccordement de cartouche et l'extrémité rétrécie de cartouche qui présente la buse.

L'enveloppe peut être soit montée à force sur le corps de cartouche, faisant alors office de frette, soit glissée à ajustement étroit sur le corps sans précontrainte préalable.

On peut aussi réaliser la cartouche en une matière plastique transparente compatible avec le produit à injecter, ce qui permettra à l'opérateur d'inspecter la dose à la partie visible et située à l'arrière de l'enveloppe métallique.

De façon avantageuse, on peut prévoir, dans l'appareil, des moyens permettant de rendre la cartouche inutilisable une fois l'éjection terminée. Ces moyens peuvent, par exemple, être portés par la tige qui pénètre dans la cartouche pour chasser le piston ou associés à cette tige pour venir rayer l'intérieur du corps de la cartouche de telle façon que celle-ci ne soit plus étanche ou venir découper ou détériorer une partie du piston lorsque celui-ci est arrivé en bout de course, supprimant ainsi l'étanchéité du piston.

L'invention a également pour objet, séparément, d'une part, l'appareil d'éjection proprement dit avec son logement, et d'autre part les cartouches conformes à l'invention.

D'autres avantages et caractéristiques de l'invention apparaîtront à la lecture de la description suivante, faite à titre d'exemple non limitatif et se référant au dessin annexé dans lequel:

la figure 1 représente une vue en coupe partielle d'une cartouche selon l'invention,

la figure 2 représente une telle cartouche dans un logement schématiquement représenté, en coupe axiale, de l'appareil.

La figure 3 représente une vue schématique en coupe d'un appareil selon l'invention.

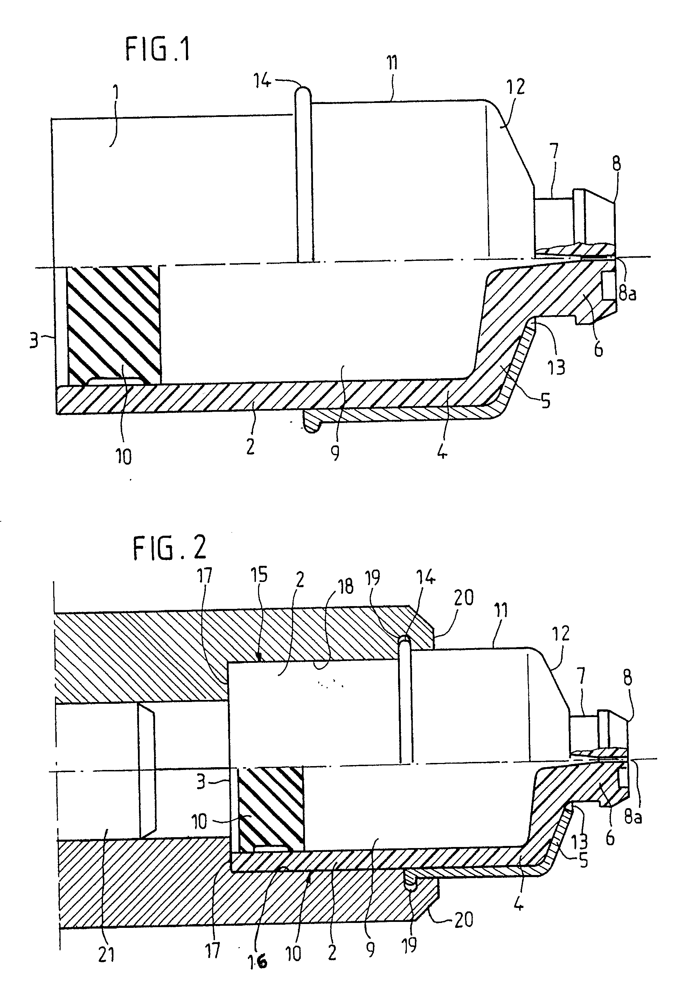

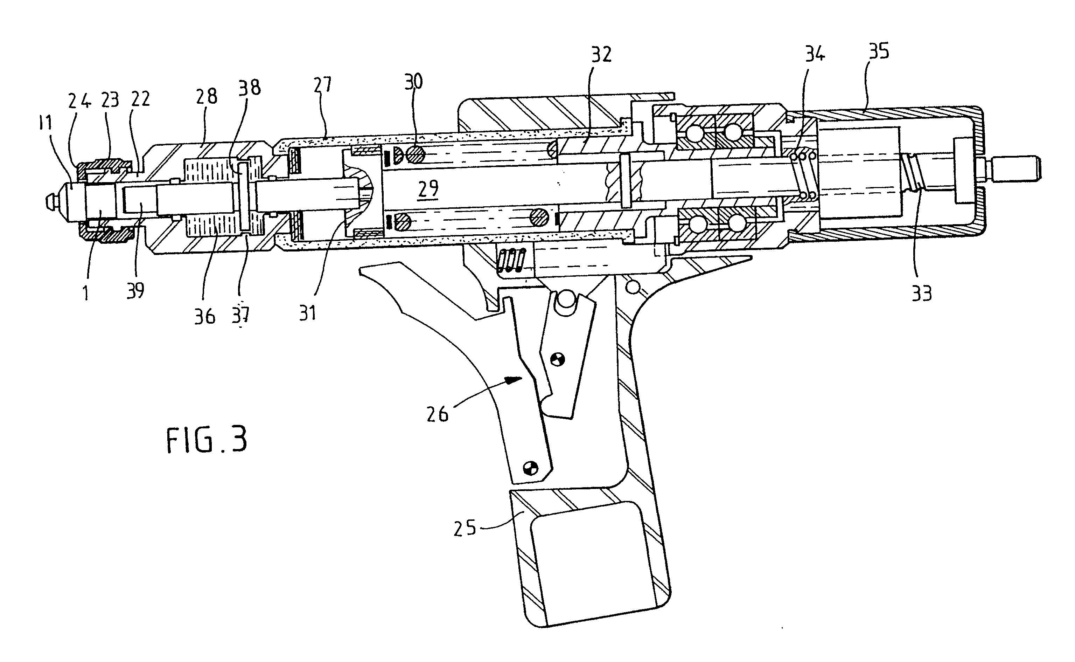

On se réfère tout d'abord aux figures 1 et 2.

La cartouche 1, conforme à l'invention, est réalisée en polypropylène. Elle comporte un corps cylindrique 2 ouvert à son extrémité postérieure 3 et refermé à son extrémité antérieure 4 par un fond 5 qui se poursuit par une extrémité antérieure rétrécie de cartouche 6 présentant une gorge 7 permettant la fixation amovible d'un capuchon usuel en caoutchouc (non représenté) destiné à garantir la stérilité de la buse d'injection 8, finement calibrée, qui débouche dans le volume 9 interne au corps 2. La dose de médicament, par exemple de vaccin, est contenue dans le volume 9 refermé à son extrémité postérieure par un piston étanche usuel 10 et l'on comprend que, lorsque le piston se trouve poussé vers l'avant par la poussée d'une tige motrice de l'appareil, elle-même actionné, au déclenchement de l'opérateur, par un organe moteur tel que, par exemple, un ressort, la dose liquide qui occupe le volume 9 est évacuée sous forme d'un jet très mince par la buse 8. Conformément à l'invention, la cartouche 1 présente une enveloppe cylindrique 11 en métal qui recouvre et entoure la plus grande partie de la moitié antérieure du corps 2. Cette enveloppe 11 présente à son extrémité antérieure un flanc 12 recouvrant la zone 5 de la cartouche et laissant en son centre un passage 13 à travers lequel a pu venir passer l'extrémité antérieure 6 de la cartouche lors de la mise en place de l'enveloppe sur le corps de cartouche. L'extrémité postérieure 14 de la cartouche présente un rebord radialement orienté vers l'extérieur.

En se référant à la figure 2, on voit la cartouche contenue dans le logement de l'appareil. L'appareil présente un logement interne constitué par deux pièces 15, 16 susceptibles d'être ramenées l'une contre l'autre autour d'un plan diamétral qui les sépare, les deux pièces 15, 16 étant de conformation interne identique. La pièce 15 fait partie d'un corps général de l'appareil alors que la pièce 16 peut avantageusement être montée sur un équipage pivotant permettant d'écarter la pièce 16 depuis la position où elle se trouve représentée sur le dessin, en direction de la flèche représentée, vers une position ouverte permettant la mise en place de la cartouche, après quoi le mouvement inverse ramène la pièce 16 dans sa position initiale de maintien dans laquelle elle complète le logement de cartouche

Ces moyens permettant d'ouvrir et de fermer un logement de cartouches d'un appareil d'administration par jet sont en soi connus et n'ont pas à être décrits de façon plus précise.

On voit que chaque demi-logement 15, 16, présente une face arrière 17 contre laquelle vient s'appliquer l'extrémité postérieure du corps de cartouche 2, une partie cylindrique 18 entourant étroitement la partie de corps de cartouche 2 qui se trouve à l'arrière de la frette ou enveloppe 11, et une demi-gorge annulaire 19 venant coiffer la nervure radiale 14, ce qui assure l'immobilisation axiale de la cartouche y compris pendant l'éjection. La face antérieure 20 des demi-logements est située à une faible distance en avant du rebord 14 et se trouve en conséquence, très éloignée du débouché 8a de la buse 8.

La cartouche étant chargée dans l'appareil, l'opérateur oriente l'appareil vers le sujet de façon que le débouché 8a de la buse 8 soit pratiquement appliqué sur la peau de la partie corporelle où doit s'effectuer l'injection. En appuyant sur une détente, il déclenche le mécanisme moteur (non représenté) de l'appareil de sorte qu'une tige 21 est propulsée en avant et, pénétrant dans le corps 2, repousse violemment le piston 10 vers l'avant et provoque l'éjection de la dose.

Ces moyens étant en soi connus, n'ont pas besoin d'être décrits plus en détail.

Les projections de matières solides et liquides, qui peuvent survenir lors de l'injection, viennent souiller l'extrémité antérieure 6 de la cartouche et, occasionnellement, la frette, surtout dans sa partie antérieure 12. En raison de la distance tant radiale qu'axiale existant entre l'extrémité antérieure 20 du logement et le débouché 8a de la buse, les extrémités 20 ne se trouvent pas souillées. Si, dans quelque circonstance, elles étaient quand même souillées, elles se trouvent de toute façon trop éloignées de l'extrémité 8a de la buse 8 de la nouvelle cartouche que l'on met en place pour le sujet suivant, pour pouvoir contaminer la buse ou la partie corporelle contre laquelle on l'applique.

L'invention peut bien entendu faire l'objet de nombreuses variantes.

En se référant à la figure 3, on voit une vue schématique d'un appareil d'administration contenant une cartouche 1 avec sa frette 11. Le logement de la partie postérieure de la cartouche 1 est pratiqué dans un embout tubulaire 22 et la cartouche est maintenue en place grâce à une douille intérieurement filetée 23 présentant un rebord antérieur 24, venant serrer contre l'extrémité correspondante de l'embout 22, la nervure 14 de la douille 11. Le vissage et le dévissage rapide de la douille 23 permet la mise en place puis l'évacuation d'une cartouche 1.

L'appareil représenté comporte une poignée 25 de type usuel avec un mécanisme de détente 26 qui ne sera pas autrement détaillée, pouvant être d'un type habituel. La poignée porte un fut 27 se prolongeant par une enceinte de freinage 28 qui porte l'embout 22. Dans le fût 27 peut coulisser une tige motrice de percussion 29 s'étendant pratiquement sur toute la longueur de l'appareil et pouvant être réalisée de façon composite. La tige 29 est sollicitée vers l'avant, c'est-à-dire dans la direction tendant à comprimer le piston de la cartouche 11 par un puissant ressort 30 logé dans le fût 27 et agissant sur un plateau 31 porté par la tige, l'arrière du ressort s'appuyant sur une pièce fixe 32.

A l'arrière de l'appareil la tige 29 présente une cannelure hélicoïdale 33 coopérant avec une cannelure périphérique correspondante 34 avec un jeu de billes interposées, cette cannelure périphérique étant susceptible d'être entrainée en rotation par une douille rotative 35 pouvant être entraînée en rotation à la main dans un sens permettant l'armement de la tige, c'est-à-dire la compression du ressort 33. Il est ainsi possible d'armer la tige en tournant la douille 35, ce qui la fait reculer et comprime le ressort 30. L'actionnement du mécanisme de détente libère la tige de sorte que le ressort 30 peut la repousser brutalement vers l'avant, le système d'encliquetage interposé entre les moyens d'armement et la tige étant d'un type usuel quelconque.

A l'extrémité antérieure du fût 30, l'enceinte 28 présente une chambre interne 36 remplie d'un fluide hydraulique, la surface interne présentant une collerette de diamètre réduit 37 au niveau de laquelle la tige 29 présente une plateau en forme de disque 38 dont la périphérie est écartée de la collerette 37 d'un très faible jeu. On comprend donc que tant que le disque 38, dans son avance avec la tige 29, n'a pas définitivement quitté la zone de la collerette 37, un freinage hydraulique important limite la vitesse d'avance de la tige 29 car le liquide hydraulique ne s'écoule pas facilement depuis l'avant du disque 38 vers l'arrière du disque. Les dimensions sont établies pour qu'au moment où le disque 38 quitte la zone de la collerette 37, l'extrémité antérieure 39 de la tige 29 vienne au contact du piston de la cartouche et à ce moment le freinage hydraulique se trouve supprimé, l'étranglement entre le disque 38 et la coleretté n'étant plus présent.

On obtient ainsi une montée brutale en pression en beaucoup moins de 1/10 de seconde, puis un plateau de pression régulier pendant toute l'éjection de la dose, la puissance du ressort assurant à la fin de l'éjection, l'éjection complète à la pression du plateau, de sorte que la quasi totalité de la dose se trouve injectée.

Il a ainsi été possible de réaliser à titre d'exemple, un dispositif extrêmement léger, ne pesant pratiquement qu'un kilo, sans pédale ni circuit hydraulique externe, facile à transporter et à entretenir et extrêmement fiable du fait de sa simplicité.

## Revendications

1. Appareil d'administration par jet sans aiguille de doses contenues dans des cartouches (1), du type comprenant un logement destiné à recevoir une cartouche (1) comprenant un corps (2) muni à son extrémité antérieure (6) d'une buse (8) permettant la formation du jet, et vers son extrémité postérieure, un piston (10) pour l'expulsion de la dose contenue dans la cartouche, ledit appareil comprenant un moyen moteur susceptible d'être déclenché par l'opérateur pour repousser vers l'avant le piston de la cartouche et assurer la formation du jet et l'évacuation de la dose, l'appareil étant agencé pour que l'extrémité antérieure (6) de la cartouche émerge de l'extrémité antérieure du logement (20) de l'appareil, suffisamment pour empêcher un contact de l'extrémité antérieure dudit logement avec la zone corporelle avec laquelle vient en contact l'extrémité de la cartouche, caractérisé en ce que la cartouche (1), réalisée en un matériau synthétique, notamment en polypropylène, présente une partie antérieure entourée par une enveloppe métallique (11) épousant étroitement ladite partie de façon à empêcher sa déformation.

2. Appareil selon la revendication 1, caractérisé en ce que ladite enveloppe joue un rôle de frette.

3. Appareil selon l'une des revendications 1 et 2, caractérisé en ce que ladite enveloppe (11) présente une partie cylindrique entourant ladite partie antérieure du corps de cartouche et une partie radiale (12) venant recouvrir le raccordement de cartouche (5) entre le diamètre extérieur de cartouche et l'extrémité rétrécie de cartouche (6) qui présente la buse.

4. Appareil selon l'une quelconque des revendications 1 à 3, caractérisé en ce que l'extrémité postérieure de ladite enveloppe (11) présente une nervure radialement orientée vers l'extérieur (14).

5. Appareil selon l'une quelconque des revendications 1 à 4, caractérisé en ce que le corps de cartouche est réalisé en matière plastique transparente laissant voir le contenu de la dose dans l'ampoule à l'arrière de ladite enveloppe.

6. Appareil selon l'une quelconque des revendications 1 à 5, caractérisé en ce qu'il comporte des moyens permettant de rendre la cartouche inutilisable une fois l'éjection terminée.

7. Appareil selon l'une quelconque des revendications 1 à 6, caractérisé en ce qu'il comporte un organe de freinage freinant le mouvement de la tige qui vient frapper le piston de la cartouche pendant la course à vide pendant laquelle l'extrémité antérieure de la tige se déplace entre sa position initiale et sensiblement la position dans laquelle elle vient au contact du piston de la cartouche.

8. Appareil selon la revendication 7, caractérisé en ce qu'il comporte un moyen moteur à ressort.

9. Appareil selon l'une quelconque des revendications 1 à 8, caractérisé en ce que l'extrémité antérieure du corps de cartouche émerge du logement sur une distance de l'ordre de 5 mm ou davantage.

10. Appareil selon la revendication 9, caractérisé en ce que la longueur de l'extrémité rétrécie (6) de la cartouche s'étendant au-delà de l'extrémité antérieure du corps de cartouche, est de l'ordre de 5 mm.

11. Appareil selon l'une des revendications 10 et 11, caractérisé en ce que le diamètre du corps de cartouche est de l'ordre de 10 mm ou davantage.

12. Cartouche en matériau synthétique comprenant un corps cylindrique, avec, à son extrémité antérieure (6) une buse (8) de formation de jet et, vers son extrémité postérieure, un piston susceptible de refouler, vers la buse, une dose à administrer, agencée pour être mise en oeuvre dans un appareil selon l'une quelconque des revendications 1 à 11, caractérisée en ce qu'elle présente une partie antérieure entourée par une enveloppe métallique (11), épousant étroitement ladite partie, de façon à empêcher sa déformation.

13. Cartouche selon l'une des revendications 12, caractérisée en ce que ladite enveloppe joue un rôle de frette.

14. Cartouche selon la revendication 12 ou 13, caractérisée en ce que ladite enveloppe (11) présente une partie cylindrique entourant ladite portée antérieure du corps de cartouche et une partie radiale (12) venant recouvrir le raccordement de cartouche (5) entre le diamètre extérieur de cartouche et l'extrémité rétrécie de cartouche (6) qui présente la buse.

15. Cartouche selon l'une quelconque des revendications 12 à 14, caractérisée en ce que l'extrémité postérieure de ladite enveloppe (11) présente une nervure radialement orientée vers l'extérieur (14).

16. Cartouche selon l'une des revendications 12 et 15, caractérisée en ce que le diamètre du corps de cartouche est de l'ordre de 10 mm ou davantage.

17. Cartouche selon l'une quelconque des revendications 12 à 16, caractérisée en ce que le corps de cartouche est réalisé en matière plastique transparente laissant voir le contenu de la dose dans l'ampoule à l'arrière de ladite enveloppe.

FIG.1

FIG.2

FIG.3

EP 0 409 674 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | US-A-3 782 380 (VAN DER GAAST) <br> * Figures 1,4; colonne 2, lignes 53-68; colonne 4, lignes 15-22 * <br> --- | 1-3,7, 10-12 | A 61 M 5/30 |
| A,D | US-A-3 688 765 (GASAWAY) <br> * Figure 2; colonne 3, lignes 54-65 * <br> --- | 1-3 | |
| A,D | US-A-2 550 053 (W.G. FERGUSON) <br> * Figure 1; colonne 2, lignes 14-21,27-33; colonne 2, ligne 55 - colonne 3, ligne 2 * <br> --- | 1,9,12 | |
| A,D | US-A-4 124 024 (SCHWEBEL et al.) <br> * Figures 1,2; colonne 2, lignes 8-12,59-65; colonne 3, lignes 1-3 * <br> --- | 1,5,9, 11,12 | |
| A,D | US-A-3 802 430 (SCHWEBEL et al.) <br> * Figure 1; colonne 2, lignes 58-60 * <br> ----- | 4 | |

DOMAINES TECHNIQUES
RECHERCHES (Int. Cl.5)

A 61 M

Le présent rapport a été établi pour toutes les revendications

| Lieu de la recherche | Date d'achèvement de la recherche | Examinateur |
|---|---|---|
| LA HAYE | 17-10-1990 | SEDY, R. |

EPO FORM 1503 03.82 (P0402)